# EUROPEAN PATENT SPECIFICATION

(11) **EP 4 520 374 B1**
(45) Date of publication and mention of the grant of the patent: **13.05.2026**
(21) Application number: 24198799.9
(22) Date of filing: 06.09.2024
(51) Int. Cl.: A61M 15/00, A61M 11/00, A61M 16/00, B05B 17/00

(54) **NEBULIZATION MEDICAMENT DELIVERY DEVICE**
VERNEBELUNGS-ARZNEIMITTELABGABEVORRICHTUNG
DISPOSITIF D'ADMINISTRATION DE MÉDICAMENT PAR NÉBULISATION

(30) Priority: 07.09.2023 US 202363536944 P; 21.03.2024 US 202463568342 P
(43) Date of publication of application: 12.03.2025
(73) Proprietor: DelBio, Inc., Taoyuan City 320023 (TW)
(72) Inventor: WU, Hung Ju, 320023 Taoyuan City (TW); LU, Yi Shen, 320023 Taoyuan City (TW)
(74) Representative: Murgitroyd & Company

(56) References cited:
- WO-A1-2019/209994
- WO-A1-2020/219012
- DE-A1- 102020 122 651
- DE-B4- 102020 110 258
- US-A1- 2017 319 796
- US-A1- 2020 353 186

## Description

### FIELD OF INVENTION

The present disclosure relates to a nebulization device, particularly relates to a nebulization medicament delivery device.

### BACKGROUND OF INVENTION

Nebulizers have been widely used in the treatment of respiratory diseases. They convert medicament solutions into a nebulized form, which can be effectively inhaled into user's lungs through a nozzle of the device. However, due to the structural design of conventional nebulizers or the lack of other additional structures (e.g., only a vent), the nebulized medicament cannot be completely inhaled into a user's body, resulting in the residue and waste of the medicament. In addition, when a user inhales, due to the uneven airflow in the nebulizer, strong pressure is laid on the user's lungs, resulting in severe discomfort. It is clear that there is still considerable room for improvement on the structure of conventional nebulizers.

DE102020110258B4 discloses an inhaler comprising a housing, an air channel extending between at least one air inlet opening and a suction opening in the housing, a dispensing element for nebulizing or vaporizing liquid supplied to the dispensing element for admixture with air flowing in the air channel, an electronic control device, an electronic data storage device, and a sensor system comprising a flow measuring device for measuring the volumetric and/or mass flow of the airflow flowing through the air channel. DE102020122651A1 discloses an inhaler, comprising a housing having a main body, the main body being configured and adapted to receive an active substance container containing an active substance, an air channel extending within the housing between at least one air inlet opening and one inhalation opening, an administration element for nebulising or vaporising the active substance fed from the active substance container to the administration element for mixing with air flowing in the air channel, an electronic control apparatus, an electronic data storage device and a sensor system having a flow measuring device for measuring the volume flow and/or mass flow of the air flowing through the air channel and/or of the active substance flowing through the air channel for storage as active substance delivery data in the electronic data storage device. WO2019209994A1 discloses an interactive apparatus for detecting and measuring inspiratory characteristic parameters of an inhalation system in use, including, inhalers and a patient's use of an inhaler in conjunction with the apparatus.

Furthermore, the flow sensing technology of dual pressure sensors is used in conventional nebulizers. The two pressure sensors are arranged inside the flow channel of the nebulizer to achieve differential pressure calculation, and the output signal of the nebulizer is a flow signal, but the disadvantage is that it is more expensive than a nebulizer using a single pressure sensor. In addition, there are also nebulizers using a single pressure sensor. However, when the sensing value of the single pressure sensor deviates in the environment, or the pressure value cannot regress properly, inaccuracy and failure may be caused, resulting in a waste of a medicament and a low dosage rate. Furthermore, the nebulizers with conventional single pressure sensor and flow sensor are expensive and large in size, requiring more space to be placed in the nebulizer.

In view of the above, it is necessary to provide a nebulization device that is different from conventional nebulizers to solve the problems existing in the conventional technology.

### SUMMARY OF INVENTION

The invention is defined in the appended claims. In order to solve the above technical problems, the present disclosure primarily provides a nebulization medicament delivery device, including:
a mouthpiece opening;
an airway;
at least one first vent configured to introduce external air into the airway;
a nebulization module including a mesh configured for nebulization;
a first pressure sensor disposed inside the nebulization medicament delivery device and to be air-tightly isolated from the airway, and configured to detect external air pressure outside the nebulization medicament delivery device;
a second pressure sensor disposed adjacent to the airway or within the airway, and configured to detect air pressure inside the airway; and
a controller,
wherein the mouthpiece opening is in communication with the at least one first vent through the airway;
wherein the controller drives the nebulization module to perform nebulization based on air pressure difference between the external air pressure and the air pressure inside the airway.

In some embodiments, the nebulization medicament delivery device further includes a medicament cup configured to contain a medicament and a detection module including a piezoelectric element.

In some embodiments, the medicament contained in the medicament cup is nebulized by the mesh, and under the condition that nebulization is being performed by the mesh, the detection module detects the pressure applied by the medicament onto the mesh through the piezoelectric element to convert the pressure into a voltage signal or a current signal, and transmits the voltage signal or the current signal to the controller for detecting an amount of the medicament contained in the medicament cup.

Preferably, the at least one first vent has a diameter of 1.5-3 mm.

In some embodiments, the nebulization medicament delivery device further includes a medicament cup cover, wherein a top of the medicament cup cover defines three holes near the mouthpiece opening arranged to improve backflow of air near the mouthpiece opening, thereby reducing residue of the medicament. Preferably, each of the three holes has a diameter of 1.4-1.8 mm. Preferably, three straight lines are formed respectively extending from the three holes to the center of the top of the medicament cup cover, and an angle between two adjacent ones of three straight lines is 30 to 50 degrees.

In some embodiments, the controller determines whether the nebulization module stops nebulization based on air pressure difference between a measurement value of the first pressure sensor and a measurement value of the second pressure sensor.

In some embodiments, under the condition that the air pressure difference between the external air pressure and the air pressure in the airway is greater than a threshold, the controller drives the nebulization module to perform nebulization. Preferably, the threshold is 60 Pa.

In some embodiments, the nebulization medicament delivery device further includes a body, wherein the first pressure sensor, the second pressure sensor and the controller are disposed inside the body, and the medicament cup cover defines the mouthpiece opening and at least part of the airway.

In some embodiments, the body and the medicament cup cover jointly define the airway, and a housing of the body defines the at least one first vent.

In some embodiments, the medicament cup cover defines the airway and the at least one first vent, and a housing of the body where the first pressure sensor is disposed defines a ventilation hole configured to allow the first pressure sensor to detect the external air pressure outside the nebulization medicament delivery device.

In some embodiments, the medicament cup cover defines the airway and the at least one first vent, and the body does not define the airway or the first vent.

In some embodiments, the space within the medicament cup cover includes a dead zone and a live zone, wherein the airway is in communication with the live zone and is not in communication with the dead zone.

In some embodiments, the at least one first vent includes two oppositely disposed vents defined by the medicament cup cover.

In some embodiments, an inner wall of the medicament cup cover protrudes radially inward to form at least one first rib in close contact with an outer wall of the medicament cup, thereby dividing space within the medicament cup cover into the dead zone and the live zone, and the dead zone and the live zone are not in communication with each other.

In some embodiments, the first rib defines a second vent, wherein the second vent penetrates the first rib, so that the mouthpiece opening is in communication with the at least one first vent through the airway and the second vent.

In some embodiments, the outer wall of the medicament cup protrudes radially outward to form at least one second rib in close contact with the first rib, thereby dividing the space within the medicament cup cover into the dead zone and the live zone, and the dead zone and the live zone are not in communication with each other.

In some embodiments, the outer wall of the medicament cup defines a third vent near bottom, wherein the third vent is in communication with the mouthpiece opening and the airway, and the third vent faces toward the second pressure sensor, so that the second pressure sensor detects the air pressure inside the airway.

Compared with conventional nebulization medicament delivery devices using a single pressure sensor or conventional nebulization medicament delivery devices using dual pressure sensors disposed inside the flow channel, in the nebulization medicament delivery device of the present disclosure, the first pressure sensor is disposed inside the nebulization medicament delivery device and is air-tightly isolated from the airway to detect the external air pressure outside the nebulization medicament delivery device, and the second pressure sensor is disposed adjacent to the airway or within the airway to detect the air pressure inside the airway. Nebulization is driven directly based on the air pressure difference between the external air pressure and the air pressure inside the airway without the need to calculate the actual flow rate, so it is more convenient and less expensive. Furthermore, the nebulization medicament delivery device of the present disclosure uses dual pressure sensors to detect the external air pressure and the air pressure inside the airway respectively. Therefore, the two pressure sensors can be calibrated with each other to prevent one of the pressure sensors from being out of accuracy and unable to regress properly, and thus nebulization cannot be performed normally.

### DESCRIPTION OF DRAWINGS

For a more complete understanding of the embodiments and their advantages, reference is now made to the following description taken in conjunction with the accompanying drawings, in which:
FIG. 1 is a schematic diagram of an internal structure of nebulization medicament delivery device 1 according to a first embodiment of the present disclosure;
FIG. 2 is a schematic diagram of an internal structure of nebulization medicament delivery device 1 according to a second embodiment of the present disclosure;
FIG. 3 is a perspective view of nebulization medicament delivery device 1 according to the second embodiment of the present disclosure;
FIGS. 4A and 4B are respectively a longitudinal section view of the A-A section of nebulization medicament delivery device 1 of FIG. 3, and a longitudinal section view of the B-B cross section of nebulization medicament delivery device 1 of FIG. 3;
FIG. 5 is a graph showing that a pressure difference between a measurement value of first pressure sensor 14 and a measurement value of second pressure sensor 15 triggers nebulization module 13 to perform nebulization;
FIG. 6 is a top view of three holes defined on a top of medicament cup cover 17 of nebulization medicament delivery device 1 of the present disclosure;
FIG. 7a shows a backflow of air when the top of medicament cup cover 17 of nebulization medicament delivery device 1 does not define any holes, and FIGS. 7b to 7d respectively show a backflow of air when the top of medicament cup cover 17 of nebulization medicament delivery device 1 defines three holes with diameters of 1.4 mm, 1.6 mm and 1.8 mm;
FIGS. 8a and 8b respectively show that when the top of medicament cup cover 17 of nebulization medicament delivery device 1 does not define any holes, there is a lot of moisture remaining near the mouthpiece opening, and after the top of medicament cup cover 17 of nebulization medicament delivery device 1 defines three holes, there is a significant reduction in moisture remaining near the mouthpiece opening;
FIGS. 9a and 9b are three-dimensional exploded views of nebulization medicament delivery device 1 according to one embodiment of the present disclosure;
FIGS. 10a to 10d are respectively a perspective view of medicament cup cover 17 of nebulization medicament delivery device 1 of the present disclosure, a bottom view of medicament cup cover 17 of nebulization medicament delivery device 1 of the present disclosure, a front view of medicament cup cover 17 of nebulization medicament delivery device 1 of the present disclosure, and a side view of medicament cup cover 17 of nebulization medicament delivery device 1 of the present disclosure;
FIG. 11 is a perspective view of medicament cup 21 of nebulization medicament delivery device 1 of the present disclosure; and
FIG. 12 is a top view of medicament cup cover 17 and medicament cup 21 of nebulization medicament delivery device 1 of the present disclosure after assembly.

### DETAILED DESCRIPTION OF PREFERRED EMBODIMENTS

Some specific embodiments according to the present disclosure will be described below; however, without departing from the scope of the present disclosure, the present disclosure can still be practiced in various forms, and the protection scope of the present disclosure should not be construed as being limited to what is stated in the specification. In addition, unless otherwise indicated in the context, "a", "the" and similar terms used in the specification (especially in the appended claims) should be understood to include both the singular and the plural forms.

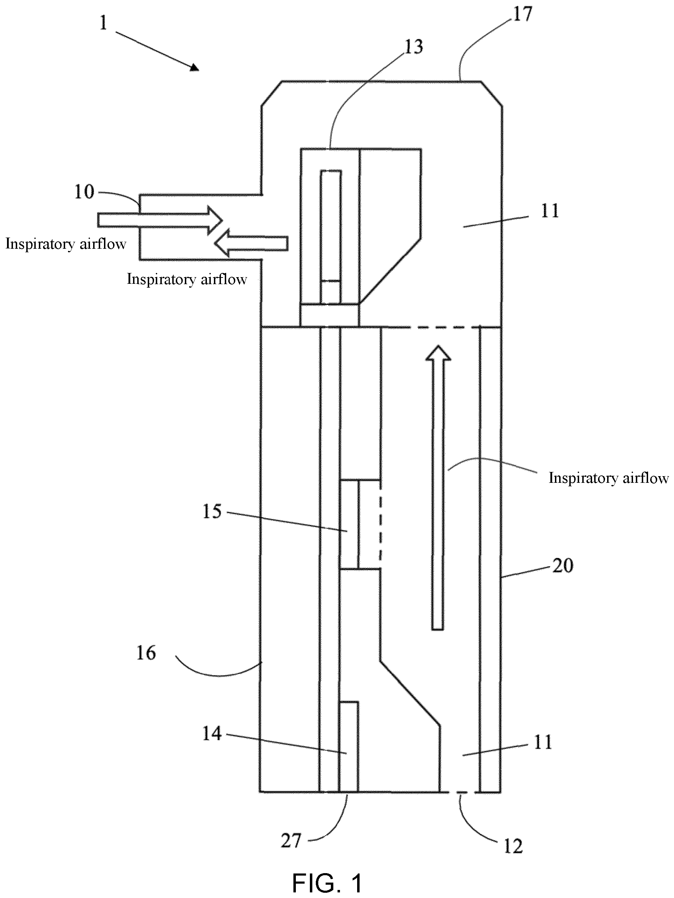

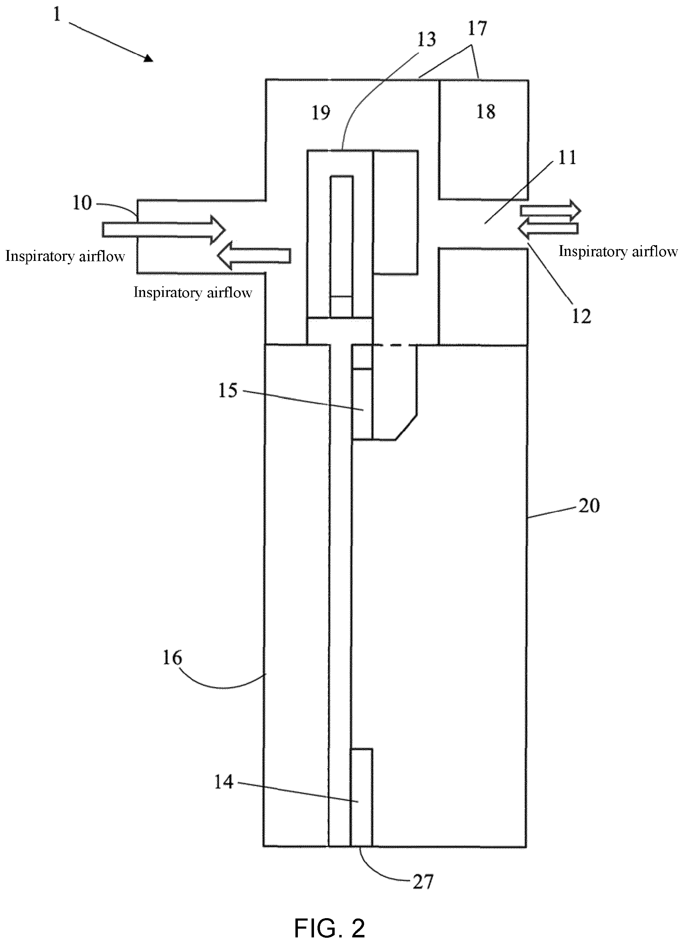

Referring to FIG. 1 and FIG. 2, one embodiment of the present disclosure provides a nebulization medicament delivery device 1 including a mouthpiece opening 10, an airway 11, at least one first vent 12, a nebulization module 13, a first pressure sensor 14, a second pressure sensor 15 and a controller (not shown). At least one first vent 12 is configured to introduce external air into airway 11. Nebulization module 13 includes a mesh and is configured for nebulization. First pressure sensor 14 is disposed inside nebulization medicament delivery device 1 and to be air-tightly isolated from airway 11, and is configured to detect external air pressure outside nebulization medicament delivery device 1. Second pressure sensor 15 is disposed adjacent to airway 11 or within airway 11, and is configured to detect air pressure inside airway 11. Mouthpiece opening 10 is in communication with at least one first vent 12 through airway 11. The controller drives nebulization module 13 to perform nebulization based on air pressure difference between the external air pressure and the air pressure inside airway 11. In an embodiment of the present disclosure, nebulization medicament delivery device 1 further includes a body 16 and a medicament cup cover 17 configured to define mouthpiece opening 10 and at least part of airway 11. In an embodiment of the present disclosure, a housing of body 16 where first pressure sensor 14 is disposed defines a ventilation hole 27 configured to allow first pressure sensor 14 to detect the external air pressure outside nebulization medicament delivery device 1.

Specifically, in the first embodiment shown in FIG. 1, airway 11 is disposed through body 16. When a user inhales through mouthpiece opening 10, the external air can be introduced into body 16 through airway 11 to form an inspiratory airflow to assist in the delivery of nebulized medicament. Body 16 and medicament cup cover 17 jointly define airway 11. It should be noted that medicament cup cover 17 does not define at least one first vent 12, and housing of the body 20 defines at least one first vent 12.

In the second embodiment shown in FIG. 2, nebulization medicament delivery device 1 further includes body 16 and medicament cup cover 17. Airway 11 is disposed through a live zone 19 of medicament cup cover 17. When a user inhales through mouthpiece opening 10, external air can be introduced into live zone 19 through airway 11 to form an inspiratory airflow to assist in the delivery of nebulized medicament. The second embodiment will be further described below.

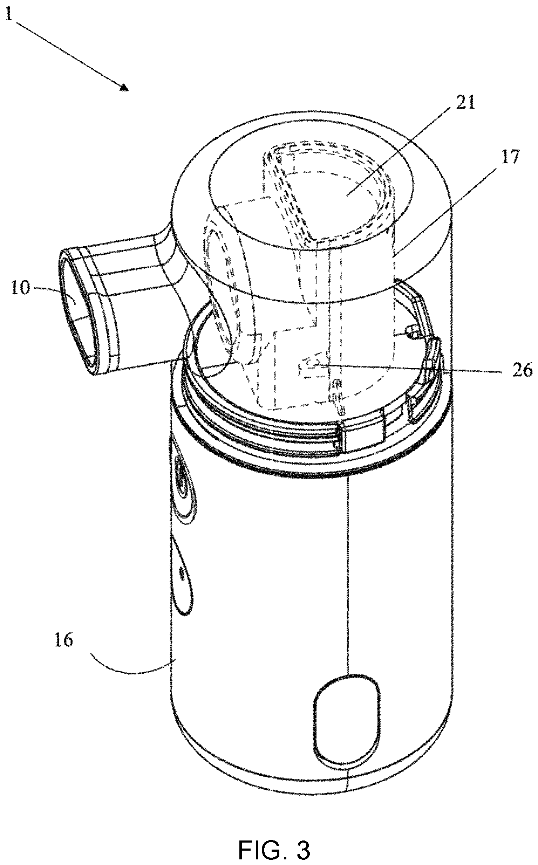

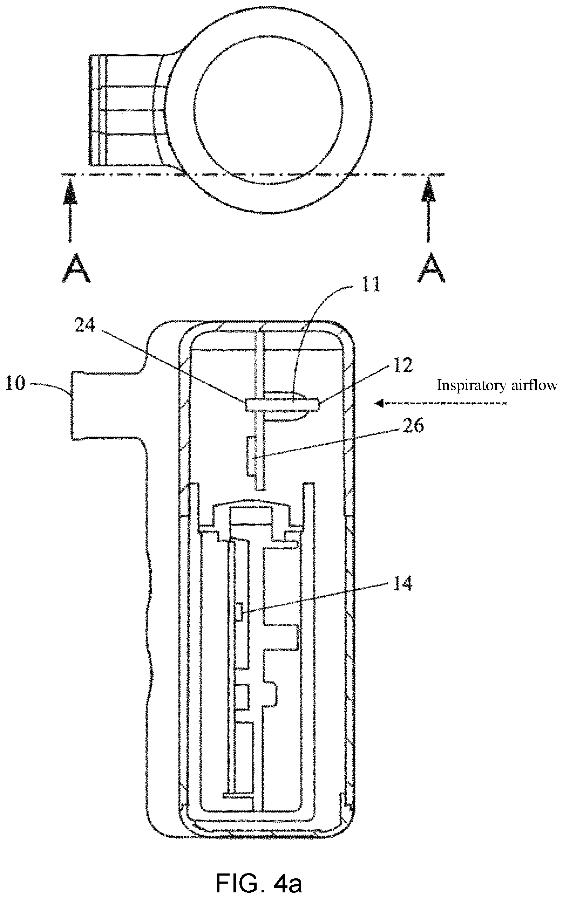

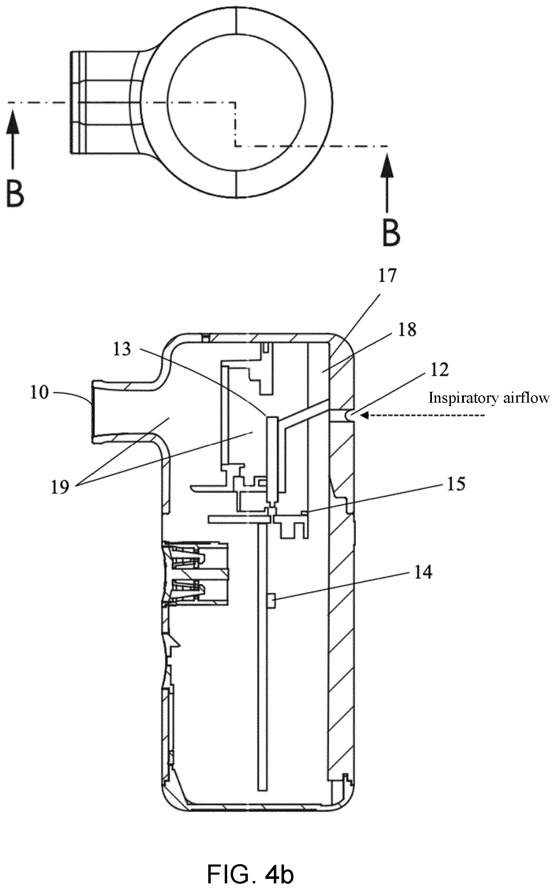

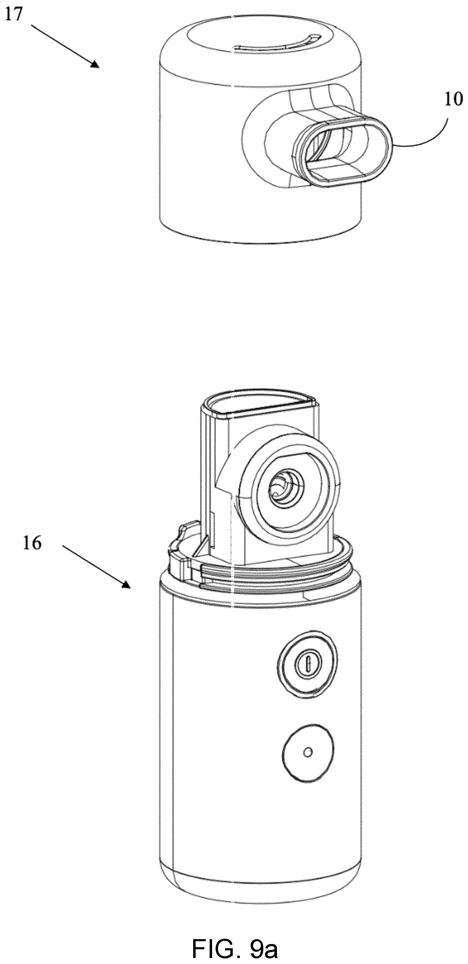

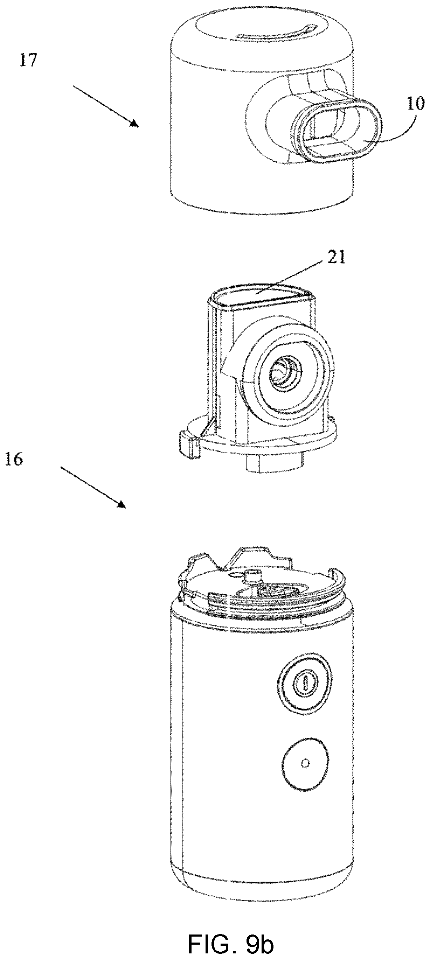

Referring to FIG. 3, FIG. 4a, FIG. 4b, FIG. 9a and FIG. 9b, in the second embodiment of the present disclosure, nebulization medicament delivery device 1 further includes a medicament cup 21 configured to contain a medicament, and a detection module (including a piezoelectric element; not shown). In the second embodiment of the present disclosure, first pressure sensor 14, second pressure sensor 15 and the controller are disposed inside body 16. In the second embodiment of the present disclosure, medicament cup cover 17 defines airway 11 and at least one first vent 12. It should be noted that body 16 does not define airway 11 or at least one first vent 12. In the preferred second embodiment of the present disclosure, the space within medicament cup cover 17 includes a dead zone 18 and a live zone 19, wherein airway 11 is in communication with live zone 19 and is not in communication with dead zone 18. The "dead zone" described herein refers to an area where there is no airflow activity, and the "live zone" described herein refers to a mix area of external airflow and the nebulized medicament. In the preferred second embodiment of the present disclosure, at least one first vent 12 includes two oppositely disposed vents defined by medicament cup cover 17.

Specifically, the external airflow is introduced into the cavity of the mouthpiece of nebulization medicament delivery device 1 through the user's inhalation, and then the air is inhaled by the human body. The internal and external pressure difference is calculated through a measurement value of first pressure sensor 14 and a measurement value of second pressure sensor 15. When the pressure difference exceeds a threshold, nebulization module 13 is driven to perform nebulization, and the nebulized medicament is absorbed by the human body. As known in the art, a negative pressure can drive the liquid to be sprayed onto the mesh together, and the high-speed impact will cause a reaction force on the mesh, causing the liquid to turn into nebulized particles and spray out. In the present disclosure, when a user holds the mouthpiece opening and inhales, the live zone forms an air-tight space and generates a negative pressure. The ratio of the dead zone to the live zone in the present disclosure may affect the negative pressure. When the live zone is smaller, the negative pressure may be generated faster and more stably, which allows the medicament to be nebulized quickly and stabilize the nebulization state. The negative pressure is also related to the diameter of the vent (which is in communication with the atmosphere). The diameter of the vent may preferably be 1.5 to 3 mm. If the diameter of the vent is too small, it may be difficult for a user to inhale. If the diameter of the vent is too large, the negative pressure may be too small and it may be difficult to achieve the nebulization effect. The airway is disposed through the dead zone but is not in communication with the dead zone. The size of the airway matches the size of the vent, and its diameter may be 1.5 to 3 mm. The smaller the diameter is, and the greater the negative pressure is, which results in a greater difference between the two pressure sensors and a higher sensitivity for triggering nebulization.

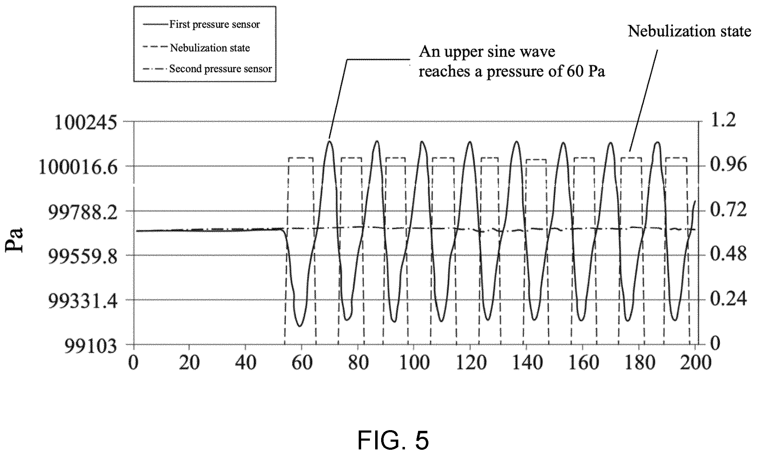

The model verification test of nebulization medicament delivery device 1 is carried out using a vent with a diameter of 1.5 mm. It can be seen from FIG. 5 that a wave pattern is generated when the flow rate of a mixed airflow is set to 5 LPM (equivalent to 0.1766 CFM). When an upper sine wave reaches a pressure of 60 Pa (i.e., the pressure between a measurement value of the first pressure sensor and a measurement value of the second pressure sensor is 60 Pa), it can trigger the mesh to perform nebulization, and the dotted line indicates the nebulization state.

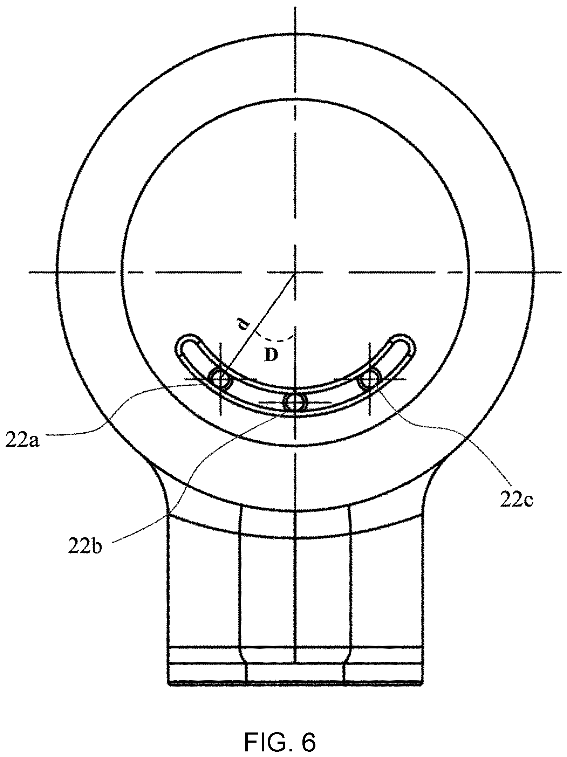

In order to reduce the backflow of air in the cavity of the mouthpiece of the nebulization medicament delivery device when a user inhales and exhales, resulting in the residue and waste of the medicament, and to reduce the pressure and discomfort on the user's lungs, the top of medicament cup cover 17 of nebulization medicament delivery device 1 of the present disclosure may be designed with three holes 22a, 22b, 22c near mouthpiece opening 10 (as shown in FIG. 6), so that the airflow can be discharged from three holes 22a, 22b, 22c, and high-pressure airflow can be introduced from mouthpiece opening 10, thereby reducing or eliminating the backflow. The diameter of each of three holes 22a, 22b, 22c may be 1.4 to 1.8 mm, and for example, may be 1.6 mm. Three straight lines are formed respectively extending from three holes 22a, 22b, 22c to the center of the top of medicament cup cover 17. The angle D between two adjacent ones of three straight lines may be 30 to 50 degrees, and, for example, may be 35 degrees. The distance d between three holes 22a, 22b, 22c and the center of the top of medicine cup cover 17 may be 12 to 20 mm, and, for example, may be 12 mm.

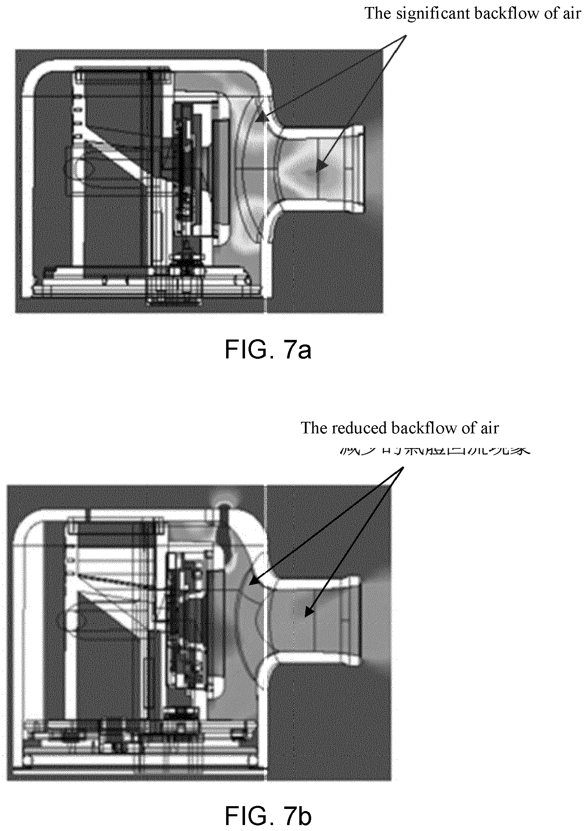

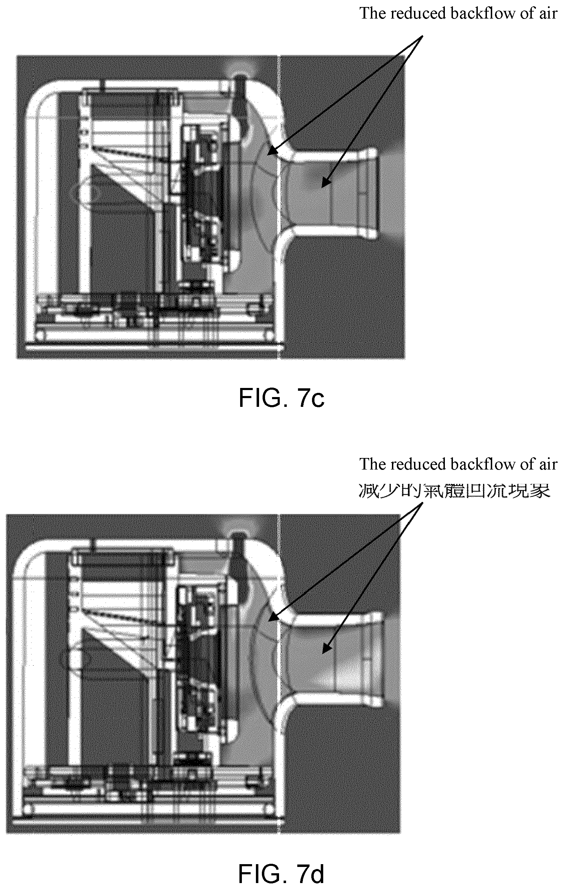

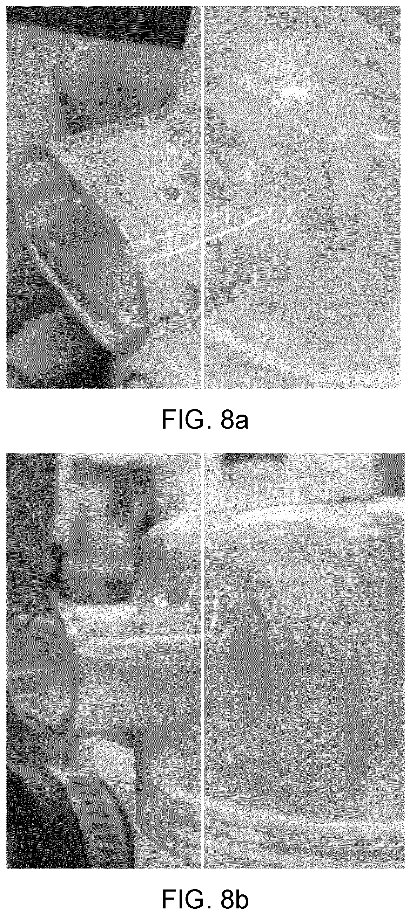

Specifically, in the model verification test conducted by the inventor of the present disclosure, when the top of medicament cup cover 17 of nebulization medicament delivery device 1 does not define any holes, the backflow of air near the cavity of the mouthpiece is significant, as shown in FIG. 7a. Under the three conditions that the top of medicament cup cover 17 of nebulization medicament delivery device 1 defines three holes 22a, 22b, 22c, each of which is 1.4 mm, 1.6 mm, and 1.8 mm respectively, the backflow of air near the cavity of the mouthpiece is significantly reduced, or even no backflow of air occurs, and the reduction or elimination of the aforementioned backflow is best achieved with a diameter of 1.6 mm, as shown in FIG. 7b to 7d. Furthermore, after defining three holes 22a, 22b, 22c on the top of medicament cup cover 17, in addition to reducing or eliminating the backflow of air, the moisture residue near the mouthpiece opening where three holes 22a, 22b, 22c are defined on the top of medicament cup cover 17 is also greatly reduced compared with the mouthpiece opening where the top of medicament cup cover 17 does not define any holes, as shown in FIG. 8a and FIG. 8b. Therefore, nebulization medicament delivery device 1 of the present disclosure can indeed effectively increase the proportion of nebulized medicament absorbed by the user, reduce the residue of the medicament, and improve the user's comfort during use.

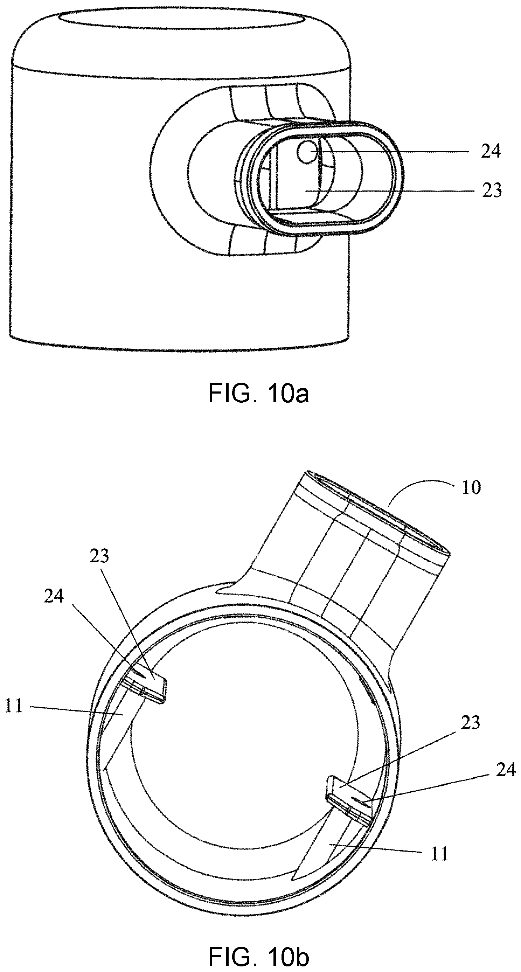

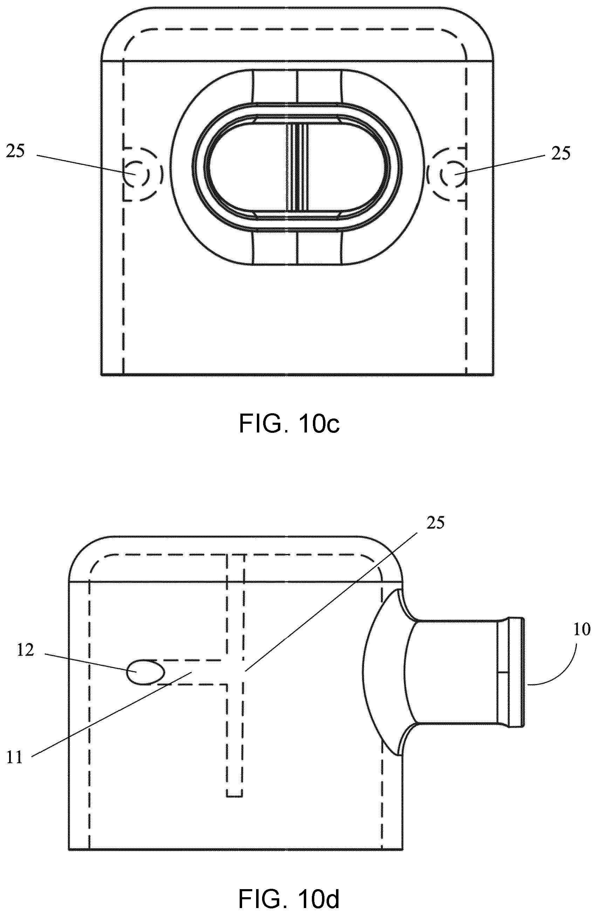

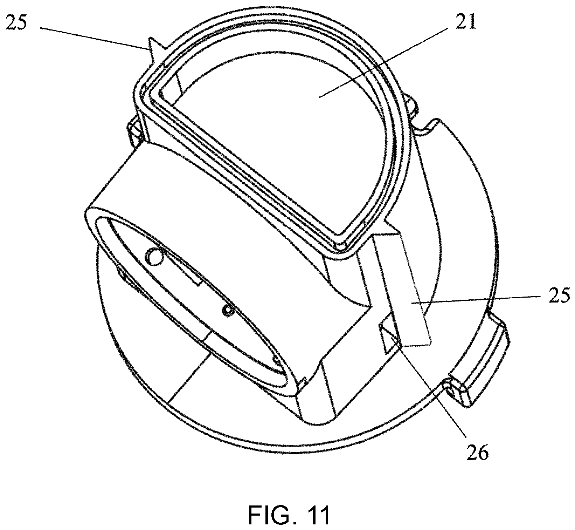

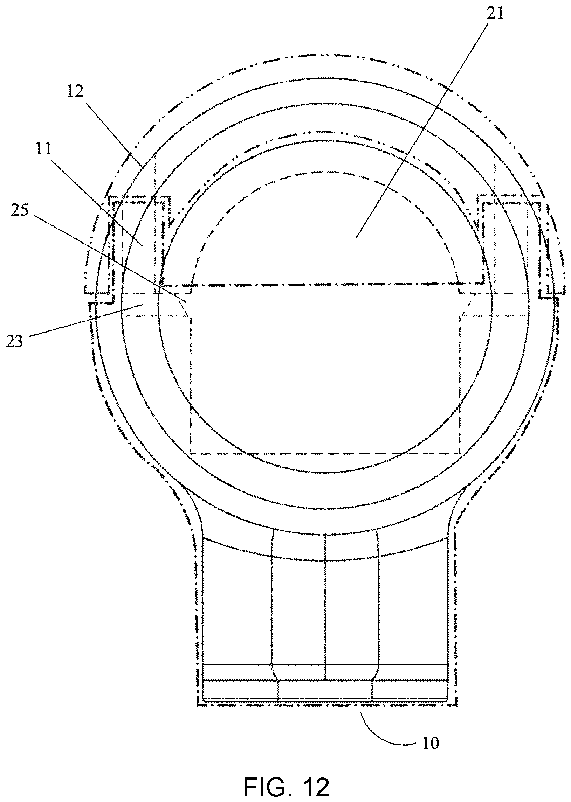

Referring to FIG. 10a to FIG. 10d, FIG. 11 and FIG. 12, in an embodiment of the present disclosure, an inner wall of medicament cup cover 17 protrudes radially inward to form at least one first rib 23 in close contact with an outer wall of medicament cup 21, thereby dividing space within medicament cup cover 17 into dead zone 18 and live zone 19, and dead zone 18 and live zone 19 are not in communication with each other. In an embodiment of the present disclosure, first rib 23 defines a second vent 24, wherein second vent 24 penetrates first rib 23, so that mouthpiece opening 10 is in communication with at least one first vent 12 through airway 11 and second vent 24. In an embodiment of the present disclosure, the outer wall of medicament cup 21 protrudes radially outward to form at least one second rib 25 in close contact with first rib 23, thereby dividing the space within medicament cup cover 17 into dead zone 18 and live zone 19, and dead zone 18 and live zone 19 are not in communication with each other (as shown in FIG. 12, the range of the one-dot chain line is live zone 19, and the range of the two-dot chain line is dead zone 18). The arrangement of first rib 23 and second rib 25 is primarily used to block the airflow and make the air tightness of the live zone better. Furthermore, first rib 23 and second rib 25 have slopes respectively to increase the contact area between medicament cup cover 17 and medicament cup 21 to facilitate disassembly and sterilization by a user.

Referring to FIG.3, FIG. 4a and FIG. 11 again, the outer wall of medicament cup 21 defines a third vent 26 near bottom, wherein third vent 26 is in communication with mouthpiece opening 10 and airway 11, and third vent 26 faces toward second pressure sensor 15, so that second pressure sensor 15 detects the air pressure inside airway 11.

Given the above, compared with conventional nebulization medicament delivery devices using a single pressure sensor or conventional nebulization medicament delivery devices using dual pressure sensors disposed inside the flow channel, in the nebulization medicament delivery device of the present disclosure, the first pressure sensor is disposed inside the nebulization medicament delivery device and is air-tightly isolated from the airway to detect the external air pressure outside the nebulization medicament delivery device, and the second pressure sensor is disposed adjacent to the airway or within the airway to detect the air pressure inside the airway. Nebulization is driven directly based on the air pressure difference between the external air pressure and the air pressure inside the airway without the need to calculate the actual flow rate, so it is more convenient and less expensive. Furthermore, the nebulization medicament delivery device of the present disclosure uses dual pressure sensors to detect the external air pressure and the air pressure inside the airway respectively. Therefore, the two pressure sensors can be calibrated with each other to prevent one of the pressure sensors from being out of accuracy and unable to regress properly, and thus nebulization cannot be performed normally.

In addition, the nebulization medicament delivery device provided by the present disclosure adjusts the structural defects of conventional nebulizers, improves the utilization rate of the medicament after nebulization and reduces the residue of the medicament, and the structural improvement of the nebulization medicament delivery device provided by the present disclosure significantly improves the airflow, so that users will not have a sense of compression when inhaling and exhaling, thereby greatly increasing the comfort during use.

## Claims

1. A nebulization medicament delivery device (1), comprising:
a mouthpiece opening (10);
an airway (11);
at least one first vent (12) configured to introduce external air into the airway (11);
a nebulization module (13) comprising a mesh configured for nebulization; a first pressure sensor (14) disposed inside the nebulization medicament delivery device (1) and to be air-tightly isolated from the airway (11), and configured to detect external air pressure outside the nebulization medicament delivery device (1);
a second pressure sensor (15) disposed adjacent to the airway (11) or within the airway (11), and configured to detect air pressure inside the airway (11); and
a controller,
**characterized in that** the nebulization medicament delivery device (1) further comprises:
a medicament cup (21) configured to contain a medicament; and
a detection module comprising a piezoelectric element;
wherein the medicament contained in the medicament cup (21) is nebulized by the mesh, and under the condition that nebulization is being performed by the mesh, the detection module detects the pressure applied by the medicament onto the mesh through the piezoelectric element to convert the pressure into a voltage signal or a current signal, and transmits the voltage signal or the current signal to the controller for detecting an amount of the medicament contained in the medicament cup (21);
wherein the mouthpiece opening (10) is in communication with the at least one first vent (12) through the airway (11);
wherein the controller drives the nebulization module (13) to perform nebulization based on air pressure difference between the external air pressure and the air pressure inside the airway (11).

2. The nebulization medicament delivery device (1) of claim 1, **characterized in that** the nebulization medicament delivery device (1) further comprises a medicament cup cover (17), wherein a top of the medicament cup (21) cover defines three holes near the mouthpiece opening (10) arranged to improve backflow of air near the mouthpiece opening (10), thereby reducing residue of the medicament.

3. The nebulization medicament delivery device (1) of claim 1, **characterized in that** the controller determines whether the nebulization module (13) stops nebulization based on air pressure difference between a measurement value of the first pressure sensor (14) and a measurement value of the second pressure sensor (15).

4. The nebulization medicament delivery device (1) of claim 1, **characterized in that** under the condition that the air pressure difference between the external air pressure and the air pressure in the airway (11) is greater than a threshold, the controller drives the nebulization module (13) to perform nebulization.

5. The nebulization medicament delivery device (1) of claim 1, **characterized in that** the nebulization medicament delivery device (1) further comprises a body (16), wherein the first pressure sensor (14), the second pressure sensor (15) and the controller are disposed inside the body (16), and the medicament cup cover (17) defines the mouthpiece opening (10) and at least part of the airway (11).

6. The nebulization medicament delivery device (1) of claim 5, **characterized in that** the medicament cup cover (17) defines the airway (11) and the at least one first vent (12), and a housing of the body (20) defines a ventilation hole (27) configured to allow the first pressure sensor (14) to detect the external air pressure outside the nebulization medicament delivery device (1).

7. The nebulization medicament delivery device (1) of claim 5, **characterized in that** the medicament cup cover (17) defines the airway (11) and the at least one first vent (12), and the body (16) does not define the airway (11) or the first vent (12).

8. The nebulization medicament delivery device (1) of claim 7, **characterized in that** the space within the medicament cup cover (17) comprises a dead zone (18) and a live zone (19), wherein the airway (11) is in communication with the live zone (19) and is not in communication with the dead zone (18).

9. The nebulization medicament delivery device of claim 7, **characterized in that** the at least one first vent (12) comprises two oppositely disposed vents defined by the medicament cup cover (17).

10. The nebulization medicament delivery device (1) of claim 8, **characterized in that** an inner wall of the medicament cup cover (17) protrudes radially inward to form at least one first rib (23) in close contact with an outer wall of the medicament cup (21), thereby dividing space within the medicament cup cover (17) into the dead zone (18) and the live zone (19), and the dead zone (18) and the live zone (19) are not in communication with each other.

11. The nebulization medicament delivery device (1) of claim 10, **characterized in that** the first rib (23) defines a second vent (24), wherein the second vent (24) penetrates the first rib (23), so that the mouthpiece opening (10) is in communication with the at least one first vent (12) through the airway (11) and the second vent (24).

12. The nebulization medicament delivery device (1) of claim 10, **characterized in that** the outer wall of the medicament cup (21) protrudes radially outward to form at least one second rib (25) in close contact with the first rib (23), thereby dividing the space within the medicament cup cover (17) into the dead zone (18) and the live zone (19), and the dead zone (18) and the live zone (19) are not in communication with each other.

13. The nebulization medicament delivery device (1) of claim 10, **characterized in that** the outer wall of the medicament cup (21) defines a third vent (26) near bottom, wherein the third vent (26) is in communication with the mouthpiece opening (10) and the airway (11), and the third vent (26) faces toward the second pressure sensor (15), so that the second pressure sensor (15) detects the air pressure inside the airway (11).

## Patentansprüche

1. Eine Medikamentenvernebelungsverabreichungsvorrichtung (1), die Folgendes beinhaltet:
eine Mundstücköffnung (10);
einen Luftweg (11);
mindestens eine erste Entlüftung (12), die konfiguriert ist, um Außenluft in den Luftweg (11) einzuführen;
ein Vernebelungsmodul (13), das ein Netz beinhaltet, das zur Vernebelung konfiguriert ist;
einen ersten Drucksensor (14), der innerhalb der
Medikamentenvernebelungsverabreichungsvorrichtung (1) angeordnet ist und luftdicht von dem Luftweg (11) zu isolieren ist und konfiguriert ist, um Außenluftdruck außerhalb der Medikamentenvernebelungsverabreichungsvorrichtung (1) zu erfassen;
einen zweiten Drucksensor (15), der angrenzend an den Luftweg (11) oder innerhalb des Luftwegs (11) angeordnet ist und konfiguriert ist, um Luftdruck innerhalb des Luftwegs (11) zu erfassen; und
eine Steuerung,
**dadurch gekennzeichnet, dass** die
Medikamentenvernebelungsverabreichungsvorrichtung (1) ferner Folgendes beinhaltet:
einen Medikamentenbecher (21), der konfiguriert ist, um ein Medikament zu enthalten;
und
ein Erfassungsmodul, das ein piezoelektrisches Element beinhaltet;
wobei das in dem Medikamentenbecher (21) enthaltene Medikament durch das Netz vernebelt wird, und unter der Bedingung, dass eine Vernebelung durch das Netz durchgeführt wird, das Erfassungsmodul den Druck erfasst, der durch das Medikament durch das piezoelektrische Element auf das Netz ausgeübt wird, um den Druck in ein Spannungssignal oder ein Stromsignal umzuwandeln, und das Spannungssignal oder das Stromsignal an die Steuerung überträgt, um eine Menge des in dem Medikamentenbecher (21) enthaltenen Medikaments zu erfassen;
wobei die Mundstücköffnung (10) mit der mindestens einen ersten Entlüftung (12) durch den Luftweg (11) in Verbindung steht;
wobei die Steuerung das Vernebelungsmodul (13) antreibt, eine Vernebelung basierend auf einer Luftdruckdifferenz zwischen dem externen Luftdruck und dem Luftdruck innerhalb des Luftwegs (11) durchzuführen.

2. Medikamentenvernebelungsverabreichungsvorrichtung (1) gemäß Anspruch 1, **dadurch gekennzeichnet, dass** die Medikamentenvernebelungsverabreichungsvorrichtung (1) ferner eine Medikamentenbecherabdeckung (17) beinhaltet, wobei eine Oberseite der Medikamentenbecher(21)-Abdeckung drei Löcher nahe der Mundstücköffnung (10) definiert, die eingerichtet sind, um den Rückfluss von Luft nahe der Mundstücköffnung (10) zu verbessern, wodurch Reste des Medikaments reduziert werden.

3. Medikamentenvernebelungsverabreichungsvorrichtung (1) gemäß Anspruch 1, **dadurch gekennzeichnet, dass** die Steuerung basierend auf einer Luftdruckdifferenz zwischen einem Messwert des ersten Drucksensors (14) und einem Messwert des zweiten Drucksensors (15) bestimmt, ob das Vernebelungsmodul (13) die Vernebelung stoppt.

4. Medikamentenvernebelungsverabreichungsvorrichtung (1) gemäß Anspruch 1, **dadurch gekennzeichnet, dass** unter der Bedingung, dass die Luftdruckdifferenz zwischen dem Außenluftdruck und dem Luftdruck in dem Luftweg (11) größer als ein Schwellenwert ist, die Steuerung das Vernebelungsmodul (13) antreibt, eine Vernebelung durchzuführen.

5. Medikamentenvernebelungsverabreichungsvorrichtung (1) gemäß Anspruch 1, **dadurch gekennzeichnet, dass** die Medikamentenvernebelungsverabreichungsvorrichtung (1) ferner einen Körper (16) beinhaltet, wobei der erste Drucksensor (14), der zweite Drucksensor (15) und die Steuerung innerhalb des Körpers (16) angeordnet sind und die Medikamentenbecherabdeckung (17) die Mundstücköffnung (10) und mindestens einen Teil des Luftwegs (11) definiert.

6. Medikamentenvernebelungsverabreichungsvorrichtung (1) gemäß Anspruch 5, **dadurch gekennzeichnet, dass** die Medikamentenbecherabdeckung (17) den Luftweg (11) und die mindestens eine erste Entlüftung (12) definiert und ein Gehäuse des Körpers (20) ein Belüftungsloch (27) definiert, das konfiguriert ist, um es dem ersten Drucksensor (14) zu ermöglichen, den Außenluftdruck außerhalb der Medikamentenvernebelungsverabreichungsvorrichtung (1) zu erfassen.

7. Medikamentenvernebelungsverabreichungsvorrichtung (1) gemäß Anspruch 5, **dadurch gekennzeichnet, dass** die Medikamentenbecherabdeckung (17) den Luftweg (11) und die mindestens eine erste Entlüftung (12) definiert und der Körper (16) den Luftweg (11) oder die erste Entlüftung (12) nicht definiert.

8. Medikamentenvernebelungsverabreichungsvorrichtung (1) gemäß Anspruch 7, **dadurch gekennzeichnet, dass** der Raum innerhalb der Medikamentenbecherabdeckung (17) eine Totzone (18) und eine Lebendzone (19) beinhaltet, wobei der Luftweg (11) mit der Lebendzone (19) in Verbindung steht und nicht mit der Totzone (18) in Verbindung steht.

9. Medikamentenvernebelungsverabreichungsvorrichtung gemäß Anspruch 7, **dadurch gekennzeichnet, dass** die mindestens eine erste Entlüftung (12) zwei gegenüberliegend angeordnete Entlüftungen beinhaltet, die durch die Medikamentenbecherabdeckung (17) definiert sind.

10. Medikamentenvernebelungsverabreichungsvorrichtung (1) gemäß Anspruch 8, **dadurch gekennzeichnet, dass** eine Innenwand der Medikamentenbecherabdeckung (17) radial nach innen vorsteht, um mindestens eine erste Rippe (23) in engem Kontakt mit einer Außenwand des Medikamentenbechers (21) zu bilden, wodurch Raum innerhalb der Medikamentenbecherabdeckung (17) in die Totzone (18) und die Lebendzone (19) unterteilt wird und die Totzone (18) und die Lebendzone (19) nicht miteinander in Verbindung stehen.

11. Medikamentenvernebelungsverabreichungsvorrichtung (1) gemäß Anspruch 10,
**dadurch gekennzeichnet, dass** die erste Rippe (23) eine zweite Entlüftung (24) definiert, wobei die zweite Entlüftung (24) die erste Rippe (23) durchdringt, sodass die Mundstücköffnung (10) mit der mindestens einen ersten Entlüftung (12) durch den Luftweg (11) und die zweite Entlüftung (24) in Verbindung steht.

12. Medikamentenvernebelungsverabreichungsvorrichtung (1) gemäß Anspruch 10,
**dadurch gekennzeichnet, dass** die Außenwand des Medikamentenbechers (21) radial nach außen vorsteht, um mindestens eine zweite Rippe (25) in engem Kontakt mit der ersten Rippe (23) zu bilden, wodurch der Raum innerhalb der Medikamentenbecherabdeckung (17) in die Totzone (18) und die Lebendzone (19) unterteilt wird und die Totzone (18) und die Lebendzone (19) nicht miteinander in Verbindung stehen.

13. Medikamentenvernebelungsverabreichungsvorrichtung (1) gemäß Anspruch 10,
**dadurch gekennzeichnet, dass** die Außenwand des Medikamentenbechers (21) eine dritte Entlüftung (26) nahe dem Boden definiert, wobei die dritte Entlüftung (26) mit der Mundstücköffnung (10) und dem Luftweg (11) in Verbindung steht und die dritte Entlüftung (26) dem zweiten Drucksensor (15) zugewandt ist, sodass der zweite Drucksensor (15) den Luftdruck innerhalb des Luftwegs (11) erfasst.

## Revendications

1. Un dispositif d'administration de médicament par nébulisation (1), comprenant :
une ouverture d'embout buccal (10) ;
un conduit d'air (11) ;
au moins un premier évent (12) configuré pour introduire de l'air externe dans le conduit d'air (11) ;
un module de nébulisation (13) comprenant un tamis configuré pour une nébulisation ;
un premier capteur de pression (14) disposé à l'intérieur du dispositif d'administration de médicament par nébulisation (1) et destiné à être isolé du conduit d'air (11) de manière étanche à l'air, et configuré pour détecter une pression d'air externe à l'extérieur du dispositif d'administration de médicament par nébulisation (1) ;
un deuxième capteur de pression (15) disposé adjacent au conduit d'air (11) ou au sein du conduit d'air (11), et configuré pour détecter une pression d'air à l'intérieur du conduit d'air (11) ; et
une unité de commande,
**caractérisé en ce que** le dispositif d'administration de médicament par nébulisation (1) comprend en outre :
un godet à médicament (21) configuré pour contenir un médicament ; et
un module de détection comprenant un élément piézoélectrique ;
dans lequel le médicament contenu dans le godet à médicament (21) est nébulisé par le tamis, et à condition qu'une nébulisation soit en train d'être effectuée par le tamis, le module de détection détecte la pression appliquée par le médicament sur le tamis par l'intermédiaire de l'élément piézoélectrique pour convertir la pression en un signal de tension ou un signal de courant, et transmet le signal de tension ou le signal de courant à l'unité de commande pour la détection d'une quantité du médicament contenu dans le godet à médicament (21) ;
dans lequel l'ouverture d'embout buccal (10) est en communication avec l'au moins un premier évent (12) par l'intermédiaire du conduit d'air (11) ;
dans lequel l'unité de commande pilote le module de nébulisation (13) pour effectuer une nébulisation sur la base d'une différence de pression d'air entre la pression d'air externe et la pression d'air à l'intérieur du conduit d'air (11).

2. Le dispositif d'administration de médicament par nébulisation (1) de la revendication 1, **caractérisé en ce que** le dispositif d'administration de médicament par nébulisation (1) comprend en outre un couvercle de godet à médicament (17), dans lequel un dessus du couvercle du godet à médicament (21) définit trois trous près de l'ouverture d'embout buccal (10) conçus pour améliorer le reflux d'air près de l'ouverture d'embout buccal (10), réduisant de ce fait un résidu du médicament.

3. Le dispositif d'administration de médicament par nébulisation (1) de la revendication 1, **caractérisé en ce que** l'unité de commande détermine si le module de nébulisation (13) arrête une nébulisation sur la base d'une différence de pression d'air entre une valeur de mesure du premier capteur de pression (14) et une valeur de mesure du deuxième capteur de pression (15).

4. Le dispositif d'administration de médicament par nébulisation (1) de la revendication 1, **caractérisé en ce qu'**à condition que la différence de pression d'air entre la pression d'air externe et la pression d'air dans le conduit d'air (11) soit supérieure à un seuil, l'unité de commande pilote le module de nébulisation (13) pour effectuer une nébulisation.

5. Le dispositif d'administration de médicament par nébulisation (1) de la revendication 1, **caractérisé en ce que** le dispositif d'administration de médicament par nébulisation (1) comprend en outre un corps (16), dans lequel le premier capteur de pression (14), le deuxième capteur de pression (15) et l'unité de commande sont disposés à l'intérieur du corps (16), et le couvercle de godet à médicament (17) définit l'ouverture d'embout buccal (10) et au moins une partie du conduit d'air (11).

6. Le dispositif d'administration de médicament par nébulisation (1) de la revendication 5, **caractérisé en ce que** le couvercle de godet à médicament (17) définit le conduit d'air (11) et l'au moins un premier évent (12), et un boîtier du corps (20) définit un trou d'aération (27) configuré pour permettre au premier capteur de pression (14) de détecter la pression d'air externe à l'extérieur du dispositif d'administration de médicament par nébulisation (1).

7. Le dispositif d'administration de médicament par nébulisation (1) de la revendication 5, **caractérisé en ce que** le couvercle de godet à médicament (17) définit le conduit d'air (11) et l'au moins un premier évent (12), et le corps (16) ne définit pas le conduit d'air (11) ni le premier évent (12).

8. Le dispositif d'administration de médicament par nébulisation (1) de la revendication 7, **caractérisé en ce que** l'espace au sein du couvercle de godet à médicament (17) comprend une zone morte (18) et une zone active (19), dans lequel le conduit d'air (11) est en communication avec la zone active (19) et n'est pas en communication avec la zone morte (18).

9. Le dispositif d'administration de médicament par nébulisation de la revendication 7, **caractérisé en ce que** l'au moins un premier évent (12) comprend deux évents disposés de manière opposée définis par le couvercle de godet à médicament (17).

10. Le dispositif d'administration de médicament par nébulisation (1) de la revendication 8, **caractérisé en ce qu'**une paroi intérieure du couvercle de godet à médicament (17) fait saillie radialement vers l'intérieur pour former au moins une première nervure (23) en contact étroit avec une paroi extérieure du godet à médicament (21), divisant de ce fait l'espace au sein du couvercle de godet à médicament (17) en la zone morte (18) et la zone active (19), et la zone morte (18) et la zone active (19) ne sont pas en communication l'une avec l'autre.

11. Le dispositif d'administration de médicament par nébulisation (1) de la revendication 10, **caractérisé en ce que** la première nervure (23) définit un deuxième évent (24), dans lequel le deuxième évent (24) pénètre la première nervure (23), de sorte que l'ouverture d'embout buccal (10) est en communication avec l'au moins un premier évent (12) par l'intermédiaire du conduit d'air (11) et du deuxième évent (24).

12. Le dispositif d'administration de médicament par nébulisation (1) de la revendication 10, **caractérisé en ce que** la paroi extérieure du godet à médicament (21) fait saillie radialement vers l'extérieur pour former au moins une deuxième nervure (25) en contact étroit avec la première nervure (23), divisant de ce fait l'espace au sein du couvercle de godet à médicament (17) en la zone morte (18) et la zone active (19), et la zone morte (18) et la zone active (19) ne sont pas en communication l'une avec l'autre.

13. Le dispositif d'administration de médicament par nébulisation (1) de la revendication 10, **caractérisé en ce que** la paroi extérieure du godet à médicament (21) définit un troisième évent (26) près du dessous, dans lequel le troisième évent (26) est en communication avec l'ouverture d'embout buccal (10) et le conduit d'air (11), et le troisième évent (26) fait face au deuxième capteur de pression (15), de sorte que le deuxième capteur de pression (15) détecte la pression d'air à l'intérieur du conduit d'air (11).
